# EUROPEAN PATENT APPLICATION

(11) **EP 1 148 089 A1**
(43) Date of publication of application: **24.10.2001**
(21) Application number: 01303447.5
(22) Date of filing: 12.04.2001
(51) Int. Cl.: C08K 5/00, A61K 7/48

(54) **Organopolysiloxane composition and method of preparation**

(30) Priority: 17.04.2000 JP 2000114704
(71) Applicant: Dow Corning Toray Silicone Co., Ltd., Tokyo (JP)
(72) Inventor: Kondo, Hidetoshi Dow Corning Toray Silicone Co.Ltd, Chiba Prefecture (JP); Takashi,Masahiro Dow Corning Toray Silicone Co.Ltd, Chiba Prefecture (JP)
(74) Representative: Kyle, Diana

(57) **Abstract**

An organopolysiloxane composition comprising (A) 5 to 99.9 wt% of a crosslinked product of organopolysiloxane which is liquid or gel-like at normal temperature containing organopolysiloxane having at least one silicon-bonded polyoxyalkylene group per molecule described by formula―R¹(OR²)ₘOR³, where R¹ and R² are independently selected alkylene groups, R³ is a group selected from the group consisting of a hydrogen atom, alkyl groups, acyl groups, and an isocyanate group, and *m* is a positive integer, and (B) 95 to 0.1 wt% of a fragrance material. The composition has superior fragrance retention capability when used to impart fragrance to other compositions such as cosmetics, detergents, and lustering agents.

## Description

The present invention relates to an organopolysiloxane composition containing a fragrance material and to a method for preparing the same, and more specifically relates to an organopolysiloxane composition possessing a superior fragrance retention capability and compounding stability, and to a method for preparing the same.

Organopolysiloxane compositions that contain fragrance materials are known and described. For example in, Japanese Application Laying Open No. Sho 61(1986)-13961 which describes a gel-like composition in which a room-temperature-curable organopolysiloxane is combined with a fragrance material, in Japanese Application Laying Open No. Hei 10(1998)-036228 which describes an organopolysiloxane composition comprising silicone rubber particles, silicone oil, and a fragrance material, and in Japanese Application Laying Open No. Hei 11(1999)-114042 which describes organopolysiloxane compositions containing silicone oil and fragrance materials.

The problem with these organopolysiloxane compositions is their insufficient fragrance retention, for example, when added to cosmetic materials, detergents, lustering agents, surface finishing agents, fiber treating agents, and coating materials.

Therefore, it is an object of the present invention to provide an organopolysiloxane composition which is superior in fragrance retention and exhibits excellent compounding stability when used as an additive for cosmetic materials, detergents, lustering agents, surface finishing agents, fiber treating agents, coating materials, etc., and furthermore which can impart excellent skin feel, finish properties, surface smoothness characteristics, surface-protecting properties, etc., to the resultant compounds, as well as to provide a method for preparing the same.

The present invention relates to an organopolysiloxane composition comprising (A) 5 to 99.9 wt% of a crosslinked product of organopolysiloxane which is liquid or gel-like at normal temperature containing organopolysiloxane having at least one silicon-bonded polyoxyalkylene group per molecule described by formula―R¹(OR²)ₘOR³, where R¹ and R² are independently selected alkylene groups, R³ is a group selected from the group consisting of a hydrogen atom, alkyl groups, acyl groups, and an isocyanate group, and *m* is a positive integer, and
(B) 95 to 0.1 wt% of a fragrance material.

The present invention is an organopolysiloxane composition comprising (A) 5 to 99.9 wt% of a crosslinked product of organopolysiloxane which is liquid or gel-like at normal temperature containing organopolysiloxane having at least one silicon-bonded polyoxyalkylene group per molecule described by formula―R¹(OR²)ₘOR³, where R¹ and R² are independently selected alkylene groups, R³ is a group selected from the group consisting of a hydrogen atom, alkyl groups, acyl groups, and an isocyanate group, and m is a positive integer, and
(B) 95 to 0.1 wt% of a fragrance material.

First of all, a detailed explanation will be provided regarding the organopolysiloxane composition of the present invention. The crosslinked product of organopolysiloxane of component (A) is characterized in that it is liquid or gel-like at normal temperature and in that it contains organopolysiloxane having at least one silicon-bonded polyoxyalkylene group per molecule described by formula―R¹(OR²)ₘOR³. In the above formula, R¹ and R² are independently selected alkylene groups exemplified by ethylene, propylene, butylene, isobutylene, pentamethylene, octamethylene, decamethylene, dodecamethylene, and cyclohexylene. Among these alkylene groups, ethylene, propylene, and butylene are preferable. R³ is selected from the group consisting of a hydrogen atom, alkyl groups, acyl groups, and an isocyanate group, where the alkyl groups are exemplified by methyl, ethyl, propyl, butyl, isobutyl, pentyl, octyl, decyl, and dodecyl; and the acyl groups are exemplified by formyl, acetyl, propionyl, butylyl, isobutylyl, valeryl, oxalyl, malonyl, succinyl, and glutaryl. The subscript *m* is a positive integer, preferably in the range of from 1 to 100 and even more preferably in the range of from 20 to 80. Polyoxyalkylenes described by formula ―R¹(OC₂H₄)ₚ(OC₃H₆)_{q}OR³ are preferable. In the formula, R¹ and R³ are the same as above. The subscript *p* is 0 or a positive integer, preferably in the range of from 0 to 20. The subscript *q* is a positive integer, preferably in the range of 20 to 80. It is necessary that *p* be smaller than *q* at all times. In addition, when *p* is a positive integer, the bond between oxyethylene and oxypropylene may be a block copolymer-type bond or a random polymer-type bond. Specific examples of the polyoxyalkylenes are described by formulas

―(CH₂)₃(OC₂H₄)₁₀(OC₃H₆)₅₀OH

―(CH₂)₃(OC₃H₆)₅₀OH

―(CH₂)₃(OC₃H₆)₃₀OCH₃

―(CH₂)₃(OC₂H₄)₅(OC₃H₆)₃₀OCH₃

―(CH₂)₂(OC₂H₄)₅(OC₃H₆)₅₀OH

―(CH₂)₂(OC₃H₆)₂₀OCOCH₃

Organopolysiloxanes with the aforementioned polyoxyalkylene groups may have a linear, partially-branched linear, branched, or cyclic molecular structure. In addition, in the organopolysiloxane, silicon-bonded groups other than the above-mentioned polyoxyalkylene groups are exemplified by substituted or unsubstituted monovalent hydrocarbon groups, such as methyl, ethyl, propyl, butyl, pentyl, hexyl, heptyl, octyl, nonyl, decyl, hexadecyl, heptadecyl, octadecyl, and other alkyl groups; cyclopentyl, cyclohexyl, and other cycloalkyl groups; phenyl, tolyl, xylyl, and other aryl groups; benzyl, phenethyl, and other aralkyl groups; and 3-chloropropyl, 3,3,3-trifluoropropyl, and other halogenated alkyl groups. In addition to these, a small amount of methoxy, ethoxy, propoxy, and other alkoxy groups and hydroxyl groups may be present.

The term "liquid," as used in the present invention with regard to the cross-linked product of the aforementioned organopolysiloxane, indicates a substance with a viscosity in the range of from 1 mPa·s to 10,000,000 mPa·s at normal temperature, with a viscosity in the range of from 100,000 mPa·s to 10,000,000 mPa·s being particularly preferred. In addition, the term "gel-like" indicates a property, whereby a substance, albeit devoid of self-flowing capacity, undergoes an irreversible deformation to a flowable liquid when an external force is applied thereto. Furthermore, the groups which are bonded to silicon atoms in the cross-linked product may comprise the same substituted or non-substituted monovalent hydrocarbons as those exemplified above for the aforementioned organopolysiloxane. In addition to these, a small amount of methoxy, ethoxy, propoxy, and other alkoxy groups and hydroxyl groups may be present.

A crosslinked product obtained by crosslinking an addition reaction-curable organopolysiloxane composition is preferable as the crosslinked product of organopolysiloxane of component (A). Specifically suggested is a crosslinked product obtained via a hydrosilation reaction between the below-described component (a), component (b), and component (c), with the crosslinked product of a crosslinkable organopolysiloxane composition comprising the below-described components (a) to (d) particularly recommended. In addition, although crosslinking is typically conducted at room temperature, if necessary it may be carried out under heating.

Component (a) is an organopolysiloxane having at least two silicon-bonded hydrogen atoms in each molecule. Component (a) is crosslinked via a hydrosilation reaction with component (c). In this organopolysiloxane, silicon-bonded groups other than hydrogen atoms are exemplified by substituted or unsubstituted monovalent hydrocarbon groups such as methyl, ethyl, propyl, butyl, pentyl, hexyl, heptyl, octyl, nonyl, decyl, hexadecyl, heptadecyl, octadecyl, and other alkyl groups; cyclopentyl, cyclohexyl, and other cycloalkyl groups; phenyl, tolyl, xylyl, and other aryl groups; benzyl, phenethyl, and other aralkyl groups; and 3-chloropropyl, 3,3,3-trifluoropropyl, and other halogenated alkyl groups. In addition to these, a small amount of methoxy, ethoxy, propoxy, and other alkoxy groups and hydroxyl groups may be present. There are no limitations concerning the structure of component (a) and suggested structures include, for example, linear, partially branched linear, branched, or cyclic structures, with linear or partially branched linear structures being preferred. There are no limitations concerning the viscosity of component (a) at 25°C; however, preferably it should be in the range of from 1 mPa·s to 100,000 mPa·s and even more preferably in the range of from 1 mPa·s to 10,000 mPa·s. In addition to dimethylpolysiloxane having both terminal ends of the molecular chain blocked by dimethylhydrogensiloxy groups, copolymer of methylhydrogensiloxane and dimethylsiloxane having both terminal ends of the molecular chain blocked by dimethylhydrogensiloxy groups, and copolymer of methylhydrogensiloxane and dimethylsiloxane having both terminal ends of the molecular chain blocked by trimethylsiloxy groups; component (a) is exemplified by organopolysiloxanes obtained by substituting phenyl, ethyl, lauryl, stearyl, 3,3,3-trifluoropropyl, etc. for some of the methyl groups in the above-mentioned polysiloxanes.

Component (b) is a characteristic component of the present invention used for improving the retention of the fragrance material in the present composition. Component (b) is an organopolysiloxane having at least one silicon-bonded polyoxyalkylene group per molecule described by formula―R¹(OR²)ₘOR³, wherein R¹, R², R³, and *m* are the same as above. The aforementioned organopolysiloxane may contain substituted or non-substituted monovalent hydrocarbon groups, other than polyoxyalkylene, bonded to silicon atoms. Such other groups can be, for example, methyl, ethyl, propyl, butyl, pentyl, hexyl, heptyl, octyl, nonyl, decyl, hexadecyl, heptadecyl, octadecyl, or a similar alkyl group; cyclopentyl, cyclohexyl, or a similar cycloalkyl group; phenyl, tolyl, xylyl, or a similar aryl group; benzyl, phenethyl, or a similar aralkyl group; and 3-chloropropyl, 3,3,3-trifluoropropyl or a similar halogenated alkyl group. In addition to the above groups, a small amount of methoxy, ethoxy, propoxy, and other alkoxy groups and hydroxyl groups may be present. The organopolysiloxane of component (b) must have no silicon-bonded alkenyl groups or silicon-bonded hydrogen atoms.

There are no limitations concerning the structure of component (b) and suggested structures include, for example, linear, partially branched linear, branched, or cyclic structures, with linear or partially branched linear structures being preferable. The viscosity of component (b) at 25°C is preferably in the range of from 10 mPa·s to 1,000,000 mPa·s and even more preferably in the range of from 100 mPa·s to 500,000 mPa·s. The amount of added component (b) is in the range of 0.1 to 100 parts by weight, preferably in the range of 0.5 to 80 parts by weight, and especially preferably in the range of 1 to 40 parts by weight per 1 part by weight of component (a). This is due to the fact that when the amount of added component (b) is less than 0.1 parts by weight, the fragrance retention capability of the present composition tends to decrease, and when it exceeds 100 parts by weight the skin feel, finish properties, surface smoothness characteristics, surface-protecting properties, and other characteristics of the resultant compounds tend to deteriorate when the present composition is added to, for example, cosmetic materials, detergents, lustering agents, surface finishing agents, fiber treating agents, and coating materials.

Examples of the aforementioned organopolysiloxane of component (b) are described by the following formulas:

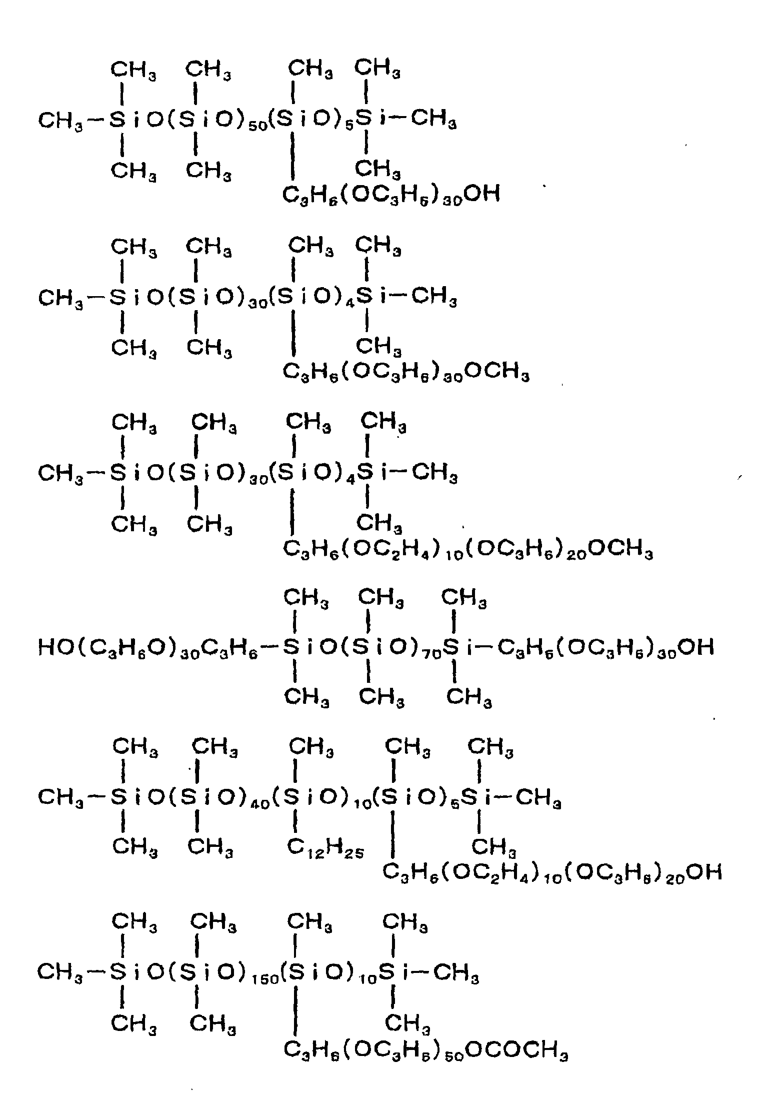

The organopolysiloxane of component (c) is crosslinked via a hydrosilation reaction with component (a). The alkenyl groups of component (c) are exemplified by vinyl, allyl, butenyl, pentenyl, and hexenyl. In this organopolysiloxane, silicon-bonded groups other than alkenyl groups are exemplified by substituted or unsubstituted monovalent hydrocarbon groups, such as methyl, ethyl, propyl, butyl, pentyl, hexyl, heptyl, octyl, nonyl, decyl, and other alkyl groups; cyclopentyl, cyclohexyl, and other cycloalkyl groups; phenyl, tolyl, xylyl, and other aryl groups; benzyl, phenethyl, and other aralkyl groups; and 3-chloropropyl, 3,3,3-trifluoropropyl, and other halogenated alkyl groups. In addition to these, a small amount of methoxy, ethoxy, propoxy, and other alkoxy groups and hydroxyl groups may be present. Suggested structures for component (c) include linear, partially branched linear, branched, or ring structures, with linear or partially branched linear structures being especially preferable. There are no limitations concerning the viscosity of component (c) at 25°C; preferably it should be in the range of from 10 mPa·s to 100,000 mPa·s, and even more preferably, in the range of from 10 mPa·s to 10,000 mPa·s. In addition to dimethylpolysiloxane having both terminal ends of the molecular chain blocked by dimethylvinylsiloxy groups, copolymer of methylvinylsiloxane and dimethylsiloxane having both terminal ends of the molecular chain blocked by dimethylvinylsiloxy groups, and copolymer of methylvinylsiloxane and dimethylsiloxane having both terminal ends of the molecular chain blocked by trimethylsiloxy groups; component (c) is exemplified by organopolysiloxanes obtained by substituting phenyl, ethyl, lauryl, stearyl, 3,3,3-trifluoropropyl, etc. for some of the methyl groups in the above-mentioned siloxanes.

The amount of added component (c) is in the range of 0.01 to 100 parts by weight, preferably, in the range of 0.1 to 70 parts by weight, and especially preferably in the range of 1 to 50 parts by weight per 1 part by weight of component (a). This is due to the fact that when the amount of added component (c) is less than 0.01 parts by weight, the skin feel, finish properties, surface smoothness characteristics, surface-protecting properties, and other characteristics of the resultant compounds tend to deteriorate to a certain extent when the present composition is added to cosmetic materials, detergents, lustering agents, surface finishing agents, fiber treating agents, coating materials, etc., and, when it exceeds 100 parts by weight the fragrance retention capability of the present composition tends to decrease.

The hydrosilation reaction catalyst of component (d) is used for promoting a hydrosilation reaction between the above-described components (a) and (c). Platinum catalysts, rhodium catalysts, and palladium catalysts are suggested as such catalysts. Among these, platinum catalysts are preferred, and specifically suggested catalysts include chloroplatinic acid, alcohol solutions of chloroplatinic acid, olefin complexes of platinum, alkenylsiloxane complexes of platinum, carbonyl complexes of platinum, platinum black, platinum catalysts supported on silica, and mixtures thereof. The amount of added component (d) is a catalytic amount. When a platinum catalyst is used the amount is preferably such that the concentration of platinum metal is within the range of 0.01 to 1,000 parts by weight per 1,000,000 parts by weight of the total of component (a) to component (c). This is due to the fact that when the platinum metal concentration is less than 0.01 parts by weight, the hydrosilation reaction does not proceed sufficiently to completion, and an amount exceeding 1,000 parts by weight does not increase the promoting the hydrosilation reaction.

Examples of component (B) include natural fragrance materials, synthetic fragrance materials, or mixed fragrance materials, which are made up of their mixtures, With account taken of solubility in the crosslinked product of organopolysiloxane, component (B) is specifically exemplified by hexanol, heptanol, octanol, nonanol, decanol, *cis*-3-hexenol, and other aliphatic alcohols; hexanal, heptanal, octanal, nonanal, decanal, 10-undecenal, and other aliphatic aldehydes; 2-octanone, methylheptenone, and other aliphatic ketones; isopentyl acetate, *cis*-3-hexenyl acetate, allyl cyclohexylpropionate, and other aliphatic esters; isoparaffin, and other aliphatic hydrocarbons; D-limonene, P-cymene and other terpene-series hydrocarbons; linalool, terpineol, citronellol, and other terpene-series alcohols; citral, citronellal, and other terpene-series aldehydes; camphor, L-carbone, menthone, and other terpene-series ketones; geranyl acetate, linalyl propionate, citronellyl isobutyrate, and other terpene-series esters; rose oxides, linalool oxides, and other terpene-series ethers; lemon oil, orange oil, lime oil, and other citrus essential oils; lavender oil, rosemary oil, peppermint oil, and other herbal essential oils; rose oil, neroli oil, and other floral essential oils; cyclopentadecanolide, and other synthetic musk fragrances. Such fragrance materials can be used singly or as a mixture of two or more materials. In addition, these fragrance materials are typically liquid at normal temperature.

The proportion, in which the above-described (A) crosslinked product of organopolysiloxane and (B) fragrance material are compounded is in the range of 5 to 99.9 : 95 to 0.1 wt%, preferably in the range of from 25 to 99 : 75 to 1 wt%, and, even more preferably in the range of 40 to 99 : 60 to 1 wt%. This is due to the fact that when the content of the fragrance material is less than 0.1 wt%, the fragrance cannot be imparted when the composition of the present invention is added to cosmetic materials, detergents, lustering agents, surface finishing agents, fiber treating agents, coating materials, and the like, and when it exceeds 95 wt% the fragrance retention capability of the present composition tends to decrease.

Although there are no limitations concerning the form of the composition of the present invention, an emulsion produced by emulsification in water is preferable. It is desirable to use a surface active agent in the preparation of the emulsion in order to improve the emulsion stability of the aforementioned crosslinkable organopolysiloxane composition. The surface active agents to be used are exemplified by anionic surface active agents, such as hexylbenzenesulfonic acid, octylbenzenesulfonic acid, decylbenzylsulfonic acid, dodecylbenzenesulfonic acid, cetylbenzenesulfonic acid, myristylbenzenesulfonic acid and their salts; cationic surface active agents, such as octyltrimethylammonium hydroxide, dodecyltrimethylammonium hydroxide, hexadecyltrimethylammonium hydroxide, octyldimethylbenzylammonium hydroxide, decyldimethylbenzylammonium hydroxide, dioctadecyldimethylammonium hydroxide, beef tallow trimethylammonium hydroxide, coconut oil trimethylammonium hydroxide; nonionic surface active agents of the polyester series, as well as ethylene oxide adduct of diethylene glycol trimethyl nonanol, polypropylene glycol, polyethylene glycol, polyoxyalkylene sorbitan ester, polyoxyalkylene alkyl ester, polyoxyalkylene alkyl phenol, and polyoxyalkylene alkyl ether. Such surface active agents can be used singly, or as a mixture of two or more agents. The use of nonionic surface active agents for emulsification is particularly preferable when using the present composition as an additive for cosmetic products.

There are no limitations concerning the amount of compounded surface active agent, but preferably it is in the range of 0.01 to 50 parts by weight, and even more preferably in the range of 0.1 to 20 parts by weight per 100 parts by weight of the present composition. This is due to the fact that when the amount is less than 0.01 parts by weight the stability of the emulsion decreases, and when it exceeds 50 parts by weight it adversely affects the characteristics of the resultant compounds when the composition is added to, for example, cosmetic materials, detergents, lustering agents, surface finishing agents, fiber treating agents, and coating materials. In addition, although there are no limitations concerning the amount of added water, preferably it is in the range of 10 to 1,000 parts by weight per 100 parts by weight of the present composition. This is due to the fact that when it is less than 10 parts by weight, the stability of the emulsion decreases, and when it exceeds 1,000 parts by weight sufficient fragrant properties cannot be imparted, for example, to cosmetic materials, detergents, lustering agents, surface finishing agents, fiber treating agents, and coating materials.

Next, a detailed description will be provided regarding the method of preparing the present organopolysiloxane composition. Examples of such methods, include a method where 5 to 99.9 wt% of a crosslinkable organopolysiloxane composition comprising the above-described components (a) to (d) and 95 to 0.1 wt% of a fragrance material are subjected to crosslinking in an emulsified state in water; and a method where the fragrance material is added after crosslinking a crosslinkable organopolysiloxane composition comprising the above-described components (a) to (d) in an emulsified state in water. In the former method, a process, in which the above-described components (a) to (c) and the fragrance material are emulsified into an emulsion in advance and then component (d) is added thereto, is preferred. It is preferable to use colloid mills, homogenizers, homomixers, and other emulsifying equipment, as well as high-shear agitators used for high-viscosity liquids. At such time, it is preferable to admix the above-described surface active agents, the use of nonionic surface active agent being especially preferable. Although the crosslinking reaction after emulsification proceeds even at room temperature, if necessary the emulsion may be heated.

In addition, in the latter method, a process in which an emulsion is prepared by emulsifying the above-described components (a) to (c) in water in advance, and then component (d) is added to the emulsion, is preferable as the process to be used for preparing the emulsion of the crosslinkable organopolysiloxane composition. It is preferable to use colloid mills, homogenizers, homomixers, and other emulsifying equipment, as well as high-shear agitators used for high-viscosity liquids. At such time, it is preferable to admix the above-described surface active agents, the use of nonionic surface active agent being especially preferable. Although the crosslinking reaction after emulsification proceeds even at room temperature, if necessary the emulsion may be heated. The fragrance material is added after preparing an aqueous emulsion of the crosslinked product of organopolysiloxane in this manner, with the mixing ratio of the crosslinked product of organopolysiloxane and the fragrance material being in the range of 5 to 99.9 : 95 to 0.1 wt%.

Because the resultant organopolysiloxane composition of the present invention is superior in fragrance retention capability when it is used as an additive for various cosmetic materials, detergents, lustering agents, surface finishing agents, fiber treating agents, coating materials, etc., the products are characterized by retaining the fragrance over an extended period of time. Furthermore, the present composition is superior in compounding stability with respect to various materials and can impart excellent skin feel, finishing properties, surface smoothness characteristics, surface-protective properties, and other characteristics to the resultant products. In addition, although the present preparative method yields an aqueous emulsion of the present composition, if necessary water can be removed.

### Application Examples

Hereinbelow, the present invention is explained in detail by referring to application examples. In the application examples, the term "viscosity" refers to a value obtained at 25°C. A liquid mixed fragrance material obtained by blending limonene, cyclohexylaldehyde, and allyl heptate in a proportion of 1:1:1 was used as the fragrance material. In addition, the fragrance retention properties, compounding stability, flexibility, and smoothness were measured in accordance with the measurement methods described below.
○ Fragrance retention properties
   A bundle of hair with a length of 15 cm and a weight of 15g was washed in an aqueous solution of sodium polyoxyethylene alkyl sulfate, rinsed, and then a test sample in the amount of 1g was applied thereto. The hair was allowed to stand indoors at a temperature of 20°C and a humidity of 40% and the presence of the scent 1 day, 2 days, and 3 days later was evaluated in the following manner.
   A: A distinct scent could be discerned.
   B: A faint scent could be discerned.
   C: No scent at all could be discerned.
○ Compounding stability
   After allowing the sample to stand for 1 day at 50°C, its appearance was visually evaluated in the following manner.
   A: Uniform.
   B: Slight separation detected.
   C: Completely separated.
○ Flexibility & Smoothness
   A bundle of hair with a length of 15 cm and a weight of 15g was washed in an aqueous solution of sodium polyoxyethylene alkyl sulfate, rinsed, and then a test sample in the amount of 1g was applied thereto. The hair was allowed to dry indoors, whereupon the flexibility and smoothness of the hair was evaluated by tactile sensation in accordance with the following evaluation criteria.
   A: Excellent.
   B: Good.
   C: Fair.
   D: Poor.

Application Example 1. A mixture was formed comprising 1.7 parts by weight of a copolymer (content of silicon-bonded hydrogen atoms: 0.8 wt%) of dimethylsiloxane and methylhydrogensiloxane having both terminal ends of the molecular chain blocked by trimethylsiloxy groups, which had a viscosity of 15 mPa·s, 5 parts by weight of an organopolysiloxane that contains polyoxypropylene described by formula 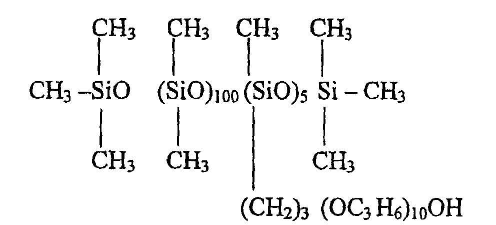 and 35.3 parts by weight of dimethylpolysiloxane (content of vinyl groups: 0.5 wt%), having both terminal ends of the molecular chain blocked by dimethylvinylsiloxy groups, the aforementioned dimethylpolysiloxane having a viscosity of 400 mPa·s, and 5 parts by weight of a fragrance material. An emulsion was prepared by adding to the mixture 3 parts by weight of polyoxyethylene lauryl ether (HLB=13.1) and 50 parts by weight of water and emulsifying. Subsequently, a solution of a 1,3-divinyltetramethyldisiloxane complex of platinum (platinum metal concentration: 0.04 wt%) was added to, and uniformly mixed with, the emulsion in an amount sufficient to produce a platinum metal concentration of 20 ppm based on the total weight of dimethylpolysiloxane, organopolysiloxane that contains polyoxypropylene groups, and the copolymer of dimethylsiloxane and methylhydrogensiloxane in the above-mentioned emulsion. Subsequently, the mixture was left to stand at room temperature for 1 day to carry out a hydrosilation reaction and prepare an emulsion of an organopolysiloxane composition. A test sample was prepared by combining 80 parts by weight of a hair conditioner (a mixture of 15 parts by weight of triethanolamine alkyl ether sulfate, 5 parts by weight of fatty acid monoethanolamide, 2 parts by weight of ethylene glycol monostearate, and 78 parts by weight of water) with 20 parts by weight of the emulsion. The fragrance retention properties, compounding stability, as well flexibility and smoothness of the sample were measured and the results are listed in Table 1. Also, a liquid with a viscosity of 1,000,000 mPa·s was obtained when water was removed from the emulsion by drying part of it at 105°C for 2 hours.

Application Example 2. A mixture comprising 1.7 parts by weight of a copolymer (content of silicon-bonded hydrogen atoms: 0.8 wt%) of dimethylsiloxane and methylhydrogensiloxane having both terminal ends of the molecular chain blocked by trimethylsiloxy groups, which had a viscosity of 15 mPa·s, 5 parts by weight of organopolysiloxane that contains polyoxypropylene groups described by formula 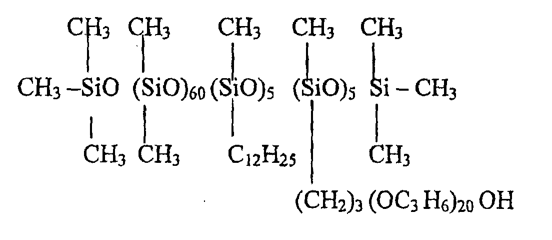 and 35.3 parts by weight of dimethylpolysiloxane (content of vinyl groups: 0.5 wt%) having both terminal ends of the molecular chain blocked by dimethylvinylsiloxy groups, which had a viscosity of 400 mPa·s. An emulsion of the mixture was prepared by adding 3 parts by weight of polyoxyethylene lauryl ether (HLB=13.1) and 50 parts by weight of water and emulsifying. Subsequently, a solution of a 1,3-divinyltetramethyldisiloxane complex of platinum (platinum metal concentration: 0.04 wt%) was added to, and uniformly mixed with, the emulsion in an amount sufficient to produce a platinum metal concentration of 20 ppm based on the total weight of dimethylpolysiloxane, organopolysiloxane that contains polyoxypropylene groups, and the copolymer of dimethylsiloxane and methylhydrogensiloxane in the above-mentioned emulsion. Subsequently, the mixture was left to stand at room temperature for 1 day to carry out a hydrosilation reaction, whereupon a white uniform emulsion of an organopolysiloxane composition was prepared by slowly adding 5 parts by weight of a fragrance material in a dropwise manner to the mixture under agitation. A test sample was prepared by combining 80 parts by weight of a hair conditioner (a mixture of 15 parts by weight of triethanolamine alkyl ether sulfate, 5 parts by weight of fatty acid monoethanolamide, 2 parts by weight of ethylene glycol monostearate, and 78 parts by weight of water) with 20 parts by weight of the emulsion. The fragrance retention properties, compounding stability, as well flexibility and smoothness of the sample were measured and the results are listed in Table 1. Also, a liquid with a viscosity of 1,000,000 mPa·s was obtained when water was removed from the emulsion by drying part of it at 105°C for 2 hours.

Comparative Example 1. An emulsion of an organopolysiloxane composition was prepared by mixing 42 parts by weight of dimethylpolysiloxane having both terminal ends of the molecular chain blocked by trimethylsiloxy groups, which had a viscosity of 1,000 mPa·s, with 5 parts by weight of a fragrance material, followed by adding 3 parts by weight of polyoxyethylene lauryl ether (HLB=13.1) and 50 parts by weight of water and emulsifying the mixture. A test sample was prepared by combining 80 parts by weight of a hair conditioner (a mixture of 15 parts by weight of triethanolamine alkyl ether sulfate, 5 parts by weight of fatty acid monoethanolamide, 2 parts by weight of ethylene glycol monostearate, and 78 parts by weight of water) with 20 parts by weight of the emulsion. The fragrance retention properties, compounding stability, as well flexibility and smoothness of the sample were measured and the results are listed in Table 1. Also, a liquid with a viscosity of 900 mPa·s was obtained when water was removed from the emulsion by drying part of it at 105°C for 2 hours.

Comparative Example 2. 1.7 Parts by weight of a copolymer (content of silicon-bonded hydrogen atoms: 0.1 wt%) of dimethylsiloxane and methylhydrogensiloxane having both terminal ends of the molecular chain blocked by trimethylsiloxy groups, which had a viscosity of 15 mPa·s, 40 parts by weight of a copolymer of dimethylsiloxane and methylvinylsiloxane (content of vinyl groups: 0.5 wt%) having both terminal ends of the molecular chain blocked by dimethylvinylsiloxy groups, which had a viscosity of 400 mPa·s, 5 parts by weight of a fragrance material, and a solution of a 1,3-divinyltetramethyldisiloxane complex of platinum, in an amount sufficient to produce a platinum metal concentration of 20 ppm based on the total weight of the above-mentioned copolymer of dimethylsiloxane and methylhydrogensiloxane and copolymer of dimethylsiloxane and methylvinylsiloxane, were combined and uniformly mixed. Subsequently, an emulsion was prepared by adding 3 parts by weight of polyoxyethylene cetyl ether (HLB=17.0) and 50 parts by weight of water and emulsifying the mixture. An emulsion of an organopolysiloxane composition was then obtained by allowing the emulsion to stand at room temperature for 1 day to carry out a hydrosilation reaction. A test sample was prepared by combining 80 parts by weight of a hair conditioner (a mixture of 15 parts by weight of triethanolamine alkyl ether sulfate, 5 parts by weight of fatty acid monoethanolamide, 2 parts by weight of ethylene glycol monostearate, and 78 parts by weight of water) with 20 parts by weight of the thus obtained emulsion. The fragrance retention properties, compounding stability, as well flexibility and smoothness of the sample were measured and the results are listed in Table 1. Also, a liquid with a viscosity of 1,000,000 mPa·s was obtained when water was removed from the emulsion by drying part of it at 105°C for 2 hours.

**Table 1**

| Examples Evaluation parameters | Application Example 1 | Application Example 2 | Comparative Example 1 | Comparative Example 2 |
|---|---|---|---|---|
| Fragrance retention properties 1 day later | A | A | B | A |
| 2 days later | A | A | C | C |
| 3 days later | B | A | C | C |
| Compounding stability | A | A | C | B |
| Flexibility | A | A | D | C |
| Smoothness | A | A | C | A |

## Claims

1. An organopolysiloxane composition comprising
(A) 5 to 99.9 wt% of a crosslinked product of organopolysiloxane which is liquid or gel-like at normal temperature containing organopolysiloxane having at least one silicon-bonded polyoxyalkylene group per molecule of formula -R¹(OR²)ₘOR³, wherein R¹ and R² are independently selected alkylene groups, R³ is a hydrogen atom, an alkyl group, acyl group or an isocyanate group, and *m* is a positive integer; and
(B) 95 to 0.1 wt% of a fragrance material.

2. An organopolysiloxane composition comprising
(A) 5 to 99.9 wt% of a crosslinked product of organopolysiloxane which is liquid or gel-like at normal temperature which is the hydrosilation reaction product of a crosslinkable organopolysiloxane composition comprising
(a) an organopolysiloxane having at least two silicon-bonded hydrogen atoms in each molecule,
(b) an organopolysiloxane having at least one silicon-bonded polyoxyalkylene group per molecule of formula -R¹(OR²)ₘOR³, wherein R¹, R² and R³ and *m* are as defined in claim 1, the organopolysiloxane being free of silicon-bonded alkenyl groups and silicon-bonded hydrogen atoms; and
(c) an organopolysiloxane having at least two silicon-bonded alkenyl groups in each molecule; and
(B) 95 to 0.1 wt% of a fragrance material.

3. The organopolysiloxane composition according to claim 1 or 2, wherein the crosslinked product of organopolysiloxane of component (A) is a crosslinked product of a crosslinkable organopolysiloxane composition comprising
1 part by weight of the organopolysiloxane (a);
0.1 to 100 parts by weight of the organopolysiloxane (b);
0.01 to 100 parts by weight of the organopolysiloxane (c); and
a catalytic amount of a hydrosilation catalyst (d).

4. The organopolysiloxane composition according to claim 2 or 3, wherein component (b) is an organopolysiloxane that contains at least one silicon-bonded polyoxyalkylene group per molecule of formula -R¹(OC₂H₄)ₚ(OC₃H₆)_{q}OR³, wherein R¹ and R³ are as defined in claim 1, *p* is 0 or a positive integer, and *q* is a positive integer, with the proviso that *p* < *q*.

5. The organopolysiloxane composition according to any of claims 1 to 4, emulsified in water.

6. The organopolysiloxane composition according to any of claims 1 to 5, wherein component (A) has a viscosity at 25°C in the range of 100,000 mPa·s to 10,000,000 mPa·s.

7. The organopolysiloxane composition according to any of claims 1 to 6, wherein the proportion of component (A) to component (B) is in the range of 40 to 99 : 60 to 1 wt%.

8. A method for preparing an organopolysiloxane composition comprising crosslinking an aqueous emulsion comprising
(A) 5 to 99.9 wt% of a crosslinkable organopolysiloxane composition comprising
(a) 1 part by weight of an organopolysiloxane having at least two silicon-bonded hydrogen atoms in each molecule,
(b) 0.1 to 100 parts by weight of an organopolysiloxane having at least one silicon-bonded polyoxyalkylene group per molecule of formula -R¹(OR²)ₘOR³, wherein R¹ and R² are independently selected alkylene groups, R³ is a hydrogen atom, an alkyl group, an acyl group or an isocyanate group, and *m* is a positive integer, the organopolysiloxane being free of silicon-bonded alkenyl groups and silicon-bonded hydrogen atoms,
(c) 0.01 to 100 parts by weight of an organopolysiloxane having at least two silicon-bonded alkenyl groups in each molecule, and
(d) a catalytic amount of a hydrosilation catalyst; and
(B) 95 to 0.1 wt% of a fragrance material.

9. The method for preparing an organopolysiloxane composition according to claim 8, wherein the crosslinking is carried out by emulsifying components (a) to (c) and the fragrance material in water and subsequently adding component (d).

10. The method for preparing an organopolysiloxane composition according to claim 8 or 9, wherein component (b) is an organopolysiloxane containing at least one silicon-bonded polyoxyalkylene group per molecule of formula -
R¹(OC₂H₄)ₚ(OC₃H₆)_{q}OR³, wherein R¹ and R² are as defined in claim 1, *p* is 0 or a positive integer, and *q* is a positive integer, with the proviso that *p*<*q*.

11. The method for preparing the organopolysiloxane composition according to any of claims 8 to 10, comprising crosslinking an aqueous emulsion of the crosslinkable organopolysiloxane composition comprising components (a) to (d) prior to addition of component (B).

12. The method for preparing an organopolysiloxane composition according to any of claims 8 to 10, wherein components (a) to (c) are emulsified in water, component (d) is added to effect crosslinking, and then component (B) is added.

13. The method for preparing an organopolysiloxane composition according to any of claims 8 to 12, wherein component (b) is an organopolysiloxane containing at least one silicon-bonded polyoxyalkylene group per molecule of formula
-R¹(OC₂H₄)ₚ(OC₃H₆)_{q}OR³, wherein R¹ and R³ are as defined in claim 1, *p* is 0 or a positive integer, and *q* is a positive integer, with the proviso that *p < q*.
